(19) Europäisches Patentamt
European Patent Office
Office européen des brevets

(11) EP 3 114 104 B1

(12) # EUROPÄISCHE PATENTSCHRIFT

(45) Veröffentlichungstag und Bekanntmachung des Hinweises auf die Patenterteilung:
**17.07.2019 Patentblatt 2019/29**

(21) Anmeldenummer: **15707978.1**

(22) Anmeldetag: **06.03.2015**

(51) Int Cl.:
***C07C 45/29*** *(2006.01)* ***C07C 49/407*** *(2006.01)*
***A61Q 11/00*** *(2006.01)* ***A61K 8/35*** *(2006.01)*

(86) Internationale Anmeldenummer:
**PCT/EP2015/054693**

(87) Internationale Veröffentlichungsnummer:
**WO 2015/132370 (11.09.2015 Gazette 2015/36)**

(54) **VERFAHREN ZUR HERSTELLUNG VON MENTHONEN AUS ISOPULEGOL IN DER GASPHASE**

METHOD FOR THE PREPARATION OF MENTHONES FROM ISOPULEGOL IN THE GAS PHASE

PROCÉDÉ DE PRÉPARATION DE MENTHONES À PARTIR DE L'ISOPULÉGOL EN PHASE GAZEUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.03.2014 EP 14158373**

(43) Veröffentlichungstag der Anmeldung:
**11.01.2017 Patentblatt 2017/02**

(60) Teilanmeldung:
**19167194.0**

(73) Patentinhaber: **BASF SE**
**67056 Ludwigshafen am Rhein (DE)**

(72) Erfinder:
- **HICKMANN, Volker**
  **67063 Ludwigshafen (DE)**
- **RÜDENAUER, Stefan**
  **69469 Weinheim (DE)**
- **DEHN, Martine**
  **67061 Ludwigshafen (DE)**
- **KELLER, Andreas**
  **67346 Speyer (DE)**
- **RENZ, Stephanie**
  **68723 Schwetzingen (DE)**
- **SCHNEIDER, Daniel**
  **67227 Frankenthal (DE)**

(74) Vertreter: **BASF IP Association**
**BASF SE**
**ZRX-C6**
**67056 Ludwigshafen (DE)**

(56) Entgegenhaltungen:
**EP-A1- 2 706 054 WO-A1-2008/016855**
**DE-A1- 4 236 111 US-A1- 2006 160 719**

- **TREIBS W ET AL: "ZUR KATALYTISCHEN DEHYDRIERUNG HYDRO-AROMATISCHER VERBINDUNGEN", CHEMISCHE BERICHTE, VCH, DE, Bd. 60, 1. Januar 1927 (1927-01-01), Seiten 2335-2341, XP008048012, ISSN: 0009-2940 in der Anmeldung erwähnt**
- **MARÍA ELENA GONZÁLEZ-NÚÑEZ ET AL: "Oxidation of Alcohols to Carbonyl Compounds with CrO 3 .SiO 2 in Supercritical Carbon Dioxide", THE JOURNAL OF ORGANIC CHEMISTRY, Bd. 71, Nr. 3, 1. Februar 2006 (2006-02-01), Seiten 1039-1042, XP055113312, ISSN: 0022-3263, DOI: 10.1021/jo052137j**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 114 104 B1

## Beschreibung

Die vorliegende Erfindung betrifft ein Verfahren zur Umsetzung von Isopulegol zu Menthon in der Gasphase und die Verwendung der so hergestellten Reaktionsprodukte als Zusatz in Lebensmitteln, Kosmetika, pharmazeutischen Erzeugnissen, Tabakformulierungen, Haushaltsprodukten und Wäschepflegeprodukten.

## Hintergrund der Erfindung

Menthol (1), der Hauptbestandteil der Kornminze (*Mentha arvensis*), gehört zu den wichtigsten Aromastoffen der Riech- und Geschmackstoffindustrie und kann in Form von vier Diastereomeren vorliegen. Die oxidierten Diastereomeren des Menthols werden als Menthon (2) bzw. Isomenthon (3) bezeichnet. Im Falle der Diastereomeren von Menthon liegen die beiden Alkylsubstituenten trans-ständig vor, bei Isomenthon sind die Substituenten *cis*-konfiguriert.
Menthon findet aufgrund seines an Pfefferminze erinnernden Geruchs- und Geschmacksprofils in vielen Formulierungen Einsatz, beispielsweise für Mundpflege und Kaugummi-Anwendungen.

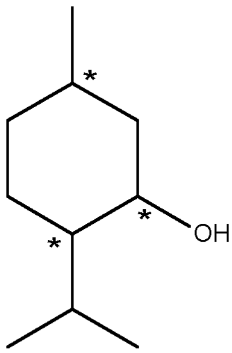
Menthol (1)

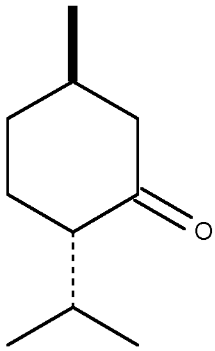
Menthon (2)

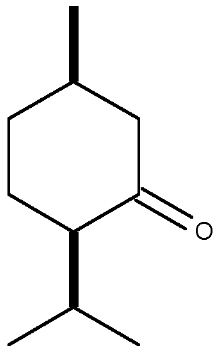
Isomenthon (3)

Üblicherweise wird Menthon oxidativ ausgehend von Menthol gewonnen. Dabei verwendete Oxidationsmittel sind Natriumchromat/Schwefelsäure (Spec. Chem. 1987, 7, 193; Acta Chem. Scand. B 1979, 33, 148), Natriumhypochlorit/Essigsäure (J. Org. Chem. 1980, 45, 2030), Ozon/Ethylacetat (JP 82180463) oder Pyridiniumchlorochromat/Silicagel (Tetrahedron 1979, 35, 1789). In der Summe sind die oxidativen Methoden zur Menthonherstellung aufgrund der Verwendung hinsichtlich des Arbeitsschutzes und der Umweltverträglichkeit bedenklicher Reagenzien nicht als befriedigend anzusehen.
Die DE 4236111 A1 beschreibt ein Verfahren zur Herstellung von Menthon aus Menthol im Festbettreaktor unter Verwendung eines heterogenen Dehydrierungskatalysators auf Kupferbasis.
W. Treibs et al. (Chem. Ber. 1927, 60, 2335) beschreiben die Umsetzung von Isopulegol (4) zu Menthon (2) in Gegenwart eines Katalysators, der aus Kupferacetat durch Fällung mit NaOH gewonnen wird.

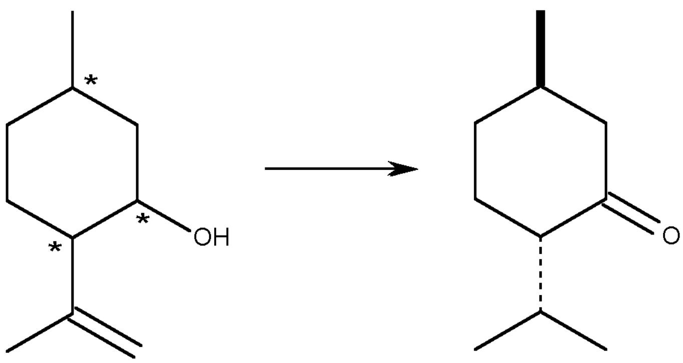
Isopulegol (4) Menthon (2)

Bei einer Reaktionstemperatur von 280 °C kann nach Überleiten des Ausgangsstoffes über den Katalysator ein Gemisch aus Menthon, Isomenthon und Thymol isoliert werden. Die darin offenbarte Reaktionsführung wird vor

allem aufgrund der umständlichen Herstellung des Katalysators und der hohen Temperaturen, die die Bildung von Nebenprodukten begünstigen, als nachteilig betrachtet.

Eine weitere Möglichkeit zur Menthonherstellung, in diesem Fall ausgehend von Citronellol (5), wird in der US 4,134,919 beschrieben.

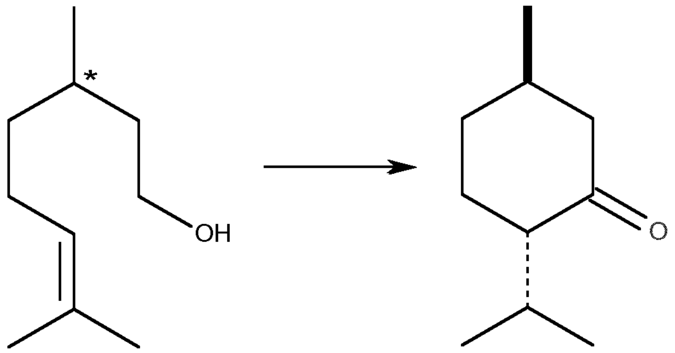

Citronellol (5) Menthon (2)

Dabei wird innerhalb von vier Stunden bei Temperaturen von 150-260 °C und kontinuierlichem Wasserstoffdruck Citronellol zu einem Menthon-/Isomenthongemisch umgesetzt. Als Katalysatoren werden Verbindungen auf Kupferbasis, z.B. Cu/Cr, Cu/Al oder Cu/Zn, eingesetzt. Isomenthon und Menthon können auch in enantiomerenangereicherter Form erhalten werden. Dabei werden Enantiomerenüberschüsse von maximal 80% erreicht.

Auch Citronellal (6) kann als Ausgangsstoff für die Menthonherstellung dienen. Forti et al. (Synthesis 2001, 1, 52) beschreiben die Cyclisierung von Citronellal in Gegenwart von Calcium-Schichtsilikaten, Aluminium- und Eisennitrat sowie einer NaOH-Lösung in Dichlorethan. Das Produktgemisch enthält Menthon und Isomenthon in einem Verhältnis von 69/31.

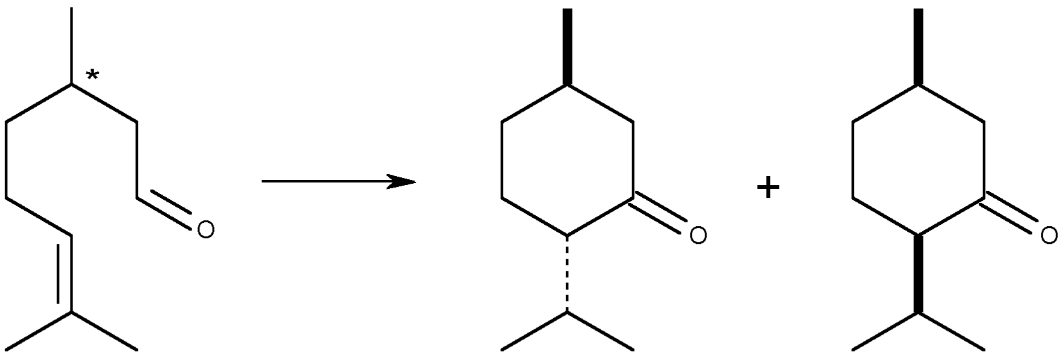

Citronellal (6) Menthon (2) Isomenthon (3)

Zusammenfassend ist festzustellen, dass die aus dem Stand der Technik bekannten Verfahren die Synthese von Menthon, auch in enantiomerenangereicherter Form, ermöglichen, jedoch die Verwendung von umwelt- oder gesundheitsschädlichen Reagenzien beinhalten. Weiterhin liefern die offenbarten Verfahren lediglich Enantiomerenüberschüsse von maximal 80%. Ferner stellen die Durchführung der Reaktion vor allem in flüssigen Reaktionsmedien und die Verwendung schwer zugänglicher Katalysatoren wesentliche Nachteile dar.

Aufgabe der Erfindung ist somit die Bereitstellung eines verbesserten Verfahrens zur katalysierten Herstellung von Menthon, bei dem insbesondere Menthon mit einem erhöhten Anteil eines Enantiomers, insbesondere enantiomerenrein, erhalten wird.

## Kurzfassung der Erfindung

Diese Aufgabe wurde durch das erfindungsgemäße Verfahren gemäß Anspruch 1 gelöst, insbesondere durch Umsetzung von Isopulegol, mit einem aktivierten oxidischen Kupferkatalysator und vor allem von Isopulegol, das einen erhöhten Anteil des Enantiomers mit (R)-Konfiguration in Position 5 aufweist, wobei Isopulegol in der Gasphase mit

einem Kupferkatalysator in Kontakt gebracht, wobei die Umsetzung bei einer Temperatur von 150-250°C erfolgt. Dabei wird vor der Umsetzung von Isopulegol der genannte Kupferkatalysator mit Wasserstoff oder insbesondere mit Wasserstoff und einem Alkohol (gleichzeitig oder zeitlich versetzt in beliebiger Reihenfolge), in einem Trägergasstrom, in Kontakt gebracht. Nach der Umsetzung wird das Reaktionsprodukt gegebenenfalls isoliert.

Ein wesentliches Produkt der Umsetzung ist Menthon, das insbesondere einen erhöhten Anteil des Enantiomers mit (*R*)-Konfiguration in Position 5 aufweist.

## Detaillierte Beschreibung der Erfindung

### a) Allgemeine Definitionen

Werden keine gegenteiligen Angaben gemacht, so umfasst "Isopulegol" die möglichen Stereoisomere:

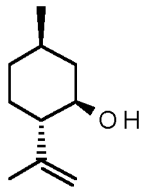

(1*R*,2*S*,5*R*)-(-)-Isopulegol

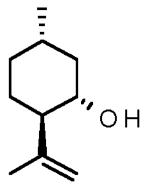

(1*S*,2*R*,5*S*)-(+)-Isopulegol

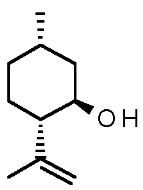

(1*R*,2*S*,5*S*)-2-Isopropenyl-5-methylcyclohexanol

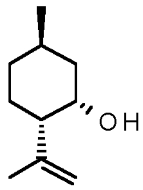

(1*S*,2*S*,5*R*)-2-Isopropenyl-5-methylcyclohexanol

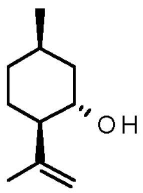

(1*S*,2*R*,5*R*)-2-Isopropenyl-5-methylcyclohexanol

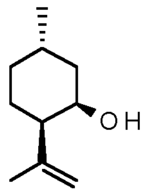

(1*R*,2*R*,5*S*)-2-Isopropenyl-5-methylcyclohexanol

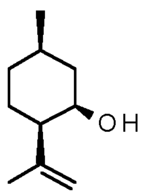

(1*R*,2*R*,5*R*)-2-Isopropenyl-5-methylcyclohexanol

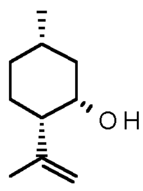

(1*S*,2*S*,5*S*)-2-Isopropenyl-5-methylcyclohexanol

Werden keine gegenteiligen Angaben gemacht, so umfasst "Menthon" die beiden Stereoisomere:

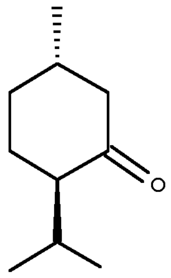 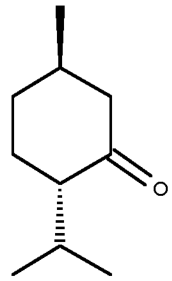

(+)-Menthon (-)-Menthon

Werden keine gegenteiligen Angaben gemacht, so umfasst "Isomenthon" die beiden Stereoisomere:

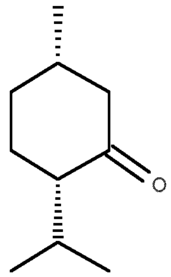 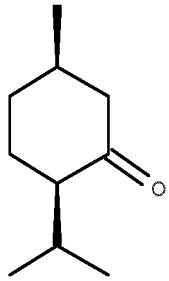

(-)-Isomenthon (+)-Isomenthon

"Enantiomerenrein" bedeutet, dass neben dem speziell benannten Enantiomer keine weitere enantiomere Form derselben chemischen Verbindung mit wenigstens einem Asymmetriezentrum analytisch nachweisbar ist.

"Enantiomerenüberschuss" gibt den Überschuss eines Enantiomers in einem Enantiomerengemisch an und wird nach folgender Formel berechnet:

$$ee = [\,|m_1 - m_2| / (m_1 + m_2)] \times 100\%$$

ee: Enantiomerenüberschuss
$m_1$ Anteil des Enantiomers 1
$m_2$: Anteil des Enantiomers 2

"Monoole" umfassen Alkanole, i.e. Alkylalkohole, und Alkenole, i.e. Alkenylalkohole, mit einer Hydroxylgruppe. Die Monoole sind primäre oder sekundäre, insbesondere primäre Monoole.

"Polyole" umfassen mehrwertige Analoga von obigen Monoolen, d.h. Alkanole, i.e. Alkylalkohole, und Alkenole, i.e. Alkenylalkohole, mit mindestens zwei, insbesondere aber mehr als zwei Hydroxylgruppen. Diole umfassen insbesondere Alkandiole, i.e. zweiwertige Analoga von Alkylalkoholen, und Alkendiole, i.e. zweiwertige Analoga von Alkenylalkoholen. Dabei sind "Alkyl" und "Alkenyl" wie unten angegeben definiert. Bevorzugte Polyole, insbesondere Diole sind solche, die wenigstens eine primäre Hydroxylgruppe umfassen. Die weiteren Hydroxylgruppen des Polyols sind insbesondere entweder sekundäre, oder besonders bevorzugt ebenfalls primäre Hydroxylgruppen.

Obige Monoole oder Polyole können bevorzugt auch als "aliphatische Polyole" bezeichnet werden.

"Alkyl" (bzw. "Alkan-" im Kontext von Alkan-Mono-oder -Polyolen) steht insbesondere für gesättigte, geradkettige oder verzweigte oder cyclische Kohlenwasserstoffreste mit 1 bis 20, 3 bis 16 oder 4 bis 12 Kohlenstoffatomen, wie z. B. Methyl, Ethyl, n-Propyl, 1-Methylethyl, n-Butyl, 1-Methyl-propyl, 2-Methylpropyl, 1,1-Dimethylethyl, n-Pentyl, 1-Methylbutyl, 2-Methylbutyl, 3-Methylbutyl, 2,2-Di-methylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl; sowie n-Heptyl, n-Octyl, n-Nonyl und n-Decyl, n-Dodecyl, n-Tetradecyl, n-Hexadecyl, n-Octadecyl, n-Eicosyl, Cyclobutyl, Cyclopentyl, Cyclohexyl, Cycloheptyl, Cyclooctyl, Cyclononyl, Cyclodecyl, Cyclododecyl, Cyclotetracyl, Cyclohexadecyl, Cyclooctadecyl, Cycloeicosyl, sowie die ein- oder mehrfach verzweigten Analoga davon.

"Alkenyl" (bzw. "Alken-" im Kontext von Alken-Mono- oder -Polyolen) steht insbesondere für die ungesättigten, geradkettigen, verzweigten oder cyclischen Analoga obiger Alkylreste und weist insbesondere 2 bis 20, 3 bis 16 oder 4 bis 12 Kohlenstoffatome auf. Insbesondere können diese ein- oder mehrfach, wie z.B. 2-, 3-, 4- oder 5-fach, insbesondere einfach ungesättigt sein. Die Doppelbindungen können dabei kumuliert, konjugiert oder nicht-konjugiert vorliegen.

"Aliphatische" Reste umfassen insbesondere nichtcyclische, geradkettige der verzweigte $C_{1-20}$- Alkyl- oder $C_{2-20}$-Alkenylreste, wie oben definiert.

Ein "Kupferkatalysator" umfasst Zusammensetzungen, die Kupfer enthalten und für die Katalyse und Aktivierung der erfindungsgemäßen Gasphasenreaktion (Umsetzung von Isopulegol zu Menthon) geeignet sind. Kupfer liegt dabei als Oxid vor. Insbesondere kann Kupfer in den Oxidationsstufen +I und +II vorliegen. Neben Kupfer können weiterhin eines oder mehrere der Elemente Aluminium, Mangan, Barium, Chrom, Calcium oder Eisen in der Katalysatorzusammensetzung vorliegen. Insbesondere können diese Elemente elementar oder als Oxide, z.B. Kupferoxid, Aluminiumoxid, Manganoxid, Bariumoxid, Chromoxid oder Eisenoxid vorliegen.

Die Elemente Aluminium, Mangan, Barium, Chrom, Calcium oder Eisen können insbesondere in den Oxidationsstufen +I bis +VI vorliegen. Weiterhin können Kombinationen der Elemente Kupfer, Aluminium, Mangan, Barium, Chrom, Calcium oder Eisen als "Doppel"- oder "Mehrfachoxide", z.B. Dichromkupfertetraoxid, Aluminiumkupferoxid ($Al_2CuO_4$), Kupferchromat, Bariumchromat, Calciumsilikat oder Palygorskit, in der Katalysatorzusammensetzung vorliegen. In den Doppel- oder Mehrfachoxiden liegen die genannten Elemente insbesondere in den Oxidationsstufen +I bis +VI vor.

Der "Kupferkatalysator" kann geträgert oder nicht geträgert vorliegen. Trägermaterialien sind z.B. Quarz, Siliciumdioxid, Aluminiumoxid oder Graphit.

Der Anteil an Kupferverbindungen im Katalysator beträgt mindestens 20 bis zu 100% bezogen auf das Gesamtgewicht des trockenen Katalysators. Insbesondere beträgt der Anteil an Kupferverbindungen im Katalysator 25 bis 95%, zum Beispiel 30-65%, insbesondere 30-45%, bezogen auf das Gesamtgewicht des trockenen Katalysators.

**b) Spezielle Ausgestaltungen**

Die vorliegende Erfindung betrifft insbesondere folgende Ausführungsformen:

1. Verfahren zur Umsetzung von Isopulegol, wobei Isopulegol in einer Trägergasphase mit einem aktivierten, oxidischen Kupferkatalysator in Kontakt gebracht wird und gegebenenfalls nach der Umsetzung ein Menthon-haltiges Reaktionsprodukt isoliert wird.

2. Verfahren nach Ausführungsform 1, wobei der Kupferkatalysator aktiviert wird, insbesondere mit Wasserstoff oder mit Wasserstoff und einem Alkohol, bevorzugt mit Wasserstoff und einem Alkohol aktiviert wird.

3. Verfahren nach einer der vorhergehenden Ausführungsformen, wobei das eingesetzte Isopulegol ein Enantiomer der Formel I umfasst, das durch (R)-Konfiguration in Position 5 gekennzeichnet ist.

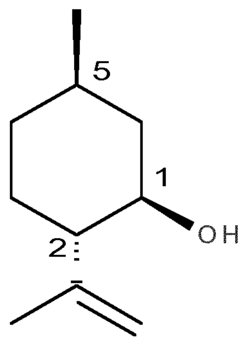

I

4. Verfahren nach einer der vorhergehenden Ausführungsformen, wobei die Reaktion bei einer Temperatur von 150-250 °C, insbesondere 160-200 °C, z.B. 170 °C, durchgeführt wird. Das Verfahren kann dabei bei 50 mBar bis 1000 mBar oder bis 1 Bar Überdruck betrieben werden. Bevorzugte Vakuumfahrweise sind 500 mBar, noch bevorzugter ist die Fahrweise bei Normaldruck.

5. Verfahren nach einer der vorhergehenden Ausführungsformen, wobei Isopulegol mit einem Katalysator auf Kupferbasis in Kontakt gebracht wird.

Dieser Kupferkatalysator umfasst Zusammensetzungen, die Kupfer enthalten und für die Katalyse und Aktivierung der erfindungsgemäßen Gasphasenreaktion geeignet sind. Kupfer liegt darin als Oxid vor. Insbesondere kann Kupfer in den Oxidationsstufen +I und +II vorliegen.

Neben Kupfer können weiterhin ein oder mehrere Elemente ausgewählt unter Aluminium, Mangan, Barium, Chrom, Calcium und Eisen in der Katalysatorzusammensetzung vorliegen. Insbesondere können diese Elemente elementar oder als Oxide, Aluminiumoxid, Manganoxid, Bariumoxid, Chromoxid oder Eisenoxid, z.B. zusammen mit Kupferoxid vorliegen.

Eine weitere Ausführungsform der Erfindung betrifft ein Verfahren zur Umsetzung von Isopulegol zu Menthon, wobei Isopulegol in einer Trägergasphase mit einem oxidischen Kupferkatalysator, gegebenenfalls enthaltend wenigstens ein weiteres Element ausgewählt unter Aluminium, Mangan, Barium, Chrom, Calcium und Eisen, in Kontakt gebracht wird und gegebenenfalls nach der Umsetzung ein Menthon-haltiges Reaktionsprodukt isoliert wird, wobei der Kupferkatalysator vor und/oder während der Umsetzung mit Wasserstoff und einem Alkohol aktiviert wird.

Die Elemente Aluminium, Mangan, Barium, Chrom, Silicium, Calcium oder Eisen können insbesondere in den Oxidationsstufen +I bis +VI vorliegen.

Weiterhin können Kombinationen der Elemente Kupfer, Aluminium, Mangan, Barium, Chrom, Calcium oder Eisen als Doppel- oder Mehrfachoxide, z.B. Dichromkupfertetraoxid, Aluminiumkupferoxid ($Al_2CuO_4$), Kupferchromat, Bariumchromat oder Palygorskit, in der Katalysatorzusammensetzung vorliegen. In den Doppel- oder Mehrfachoxiden liegen die genannten Elemente insbesondere in den Oxidationsstufen +I bis +VI vor.

Vorzugsweise ist die oxidische Kupferkomponente der Katalysatorzusammensetzung Kupferoxid. Vorzugsweise sind die, gegebenenfalls zusätzlich enthaltenen, katalytisch aktiven Komponenten der Katalysatorzusammensetzung ausgewählt unter den Verbindungen Aluminiumoxid, Manganoxid, Aluminiumkupferoxid, Chromkupferoxid, Bariumoxid, Dichromtrioxid, Chromtrioxid, Kupferchromat, Bariumchromat, Calciumsilikat und Palygorskit und Mischungen davon, insbesondere Mischungen enthaltend 2, 3, 4 oder 5 verschiedene Verbindungen davon. Insbesondere bevorzugt sind Zusammensetzungen umfassend Kupferoxid, Aluminiumoxid, Manganoxid und Aluminiumkupferoxid.

Der Kupferkatalysator kann geträgert oder nicht geträgert vorliegen. Trägermaterialien sind z.B. Quartz, Siliciumdioxid, Aluminiumoxid oder Graphit.

Der Anteil an Kupferverbindungen im Katalysator beträgt mindestens 20 bis zu 100% bezogen auf das Gesamtgewicht des trockenen Katalysators. Insbesondere beträgt der Anteil an Kupferverbindungen im Katalysator 25 bis 95%, z.B. 30-65%, insbesondere 30-45%, bezogen auf das Gesamtgewicht des trockenen Katalysators.

In einer Ausführungsform der Erfindung weist der Katalysator eine Zusammensetzung umfassend 30-40% Kupferoxid, 10-25% Aluminiumoxid, 10-25% Manganoxid und 30-40% Aluminiumkupferoxid, jeweils bezogen auf die Gesamtmasse des trockenen Katalysators, auf.

Weitere Beispiele für erfindungsgemäß bevorzugte Kupferkatalysatoren umfassen die Handelsprodukte X 540 T 1/8, E 406 T 1/8, Cu 1986 T 1/8, Cu 1808 T 1/8, Cu 1230 E1/16 oder Cu 0865 T 3/16 der BASF Corporation (Florham Park, NJ 07932, USA) mit den folgenden Zusammensetzungen:

**X 540 T 1/8***
30.0-45.0 Gew.-% Kupferoxid
30.0-40.0 Gew.-% Aluminiumkupferoxid ($Al_2CuO_4$)
10.0-25.0 Gew.-% Aluminiumoxid
10.0-20.0 Gew.-% Mangandioxid

**E 406 T 1/8***
60.0-65.0 Gew.-% Chromkupferoxid ($Cr_2CuO_4$)
20.0-25.0 Gew.-% Kupferoxid
5.0-10.0 Gew.-% Bariumoxid
1.0-5.0 Gew.-% Graphit
1.0 Gew.-% Dichromtrioxid
1.0 Gew.-% Chromtrioxid

**Cu 1986 T 1/8***
60.0-70.0 Gew.-% Chromkupferoxid ($Cr_2CuO_4$)
20.0-30.0 Gew.-% Kupferoxid
1.0-5.0 Gew.-% Mangandioxid
1.0-5.0 Gew.-% Kieselsäure, Natriumsalz

1.0-5.0 Gew.-% Graphit
0.0-0.5 Gew.-% Kupferchromat

**Cu 1808 T 1/8***
55.0-65.0 Gew.-% Chromkupferoxid ($Cr_2CuO_4$)
20.0-30.0 Gew.-% Kupferoxid
5.0-10.0 Gew.-% Siliciumdioxid
5.0-10.0 Gew.-% Kieselsäure, Natriumsalz
1.0-5.0 Gew.-% Graphit

**Cu 1230 E 1/16***
41.0-46.0 Gew.-% Chromkupferoxid ($Cr_2CuO_4$)
25.0-35.0 Gew.-% Aluminiumoxid
13.0-17.0 Gew.-% Kupferoxid
10.0-13.0 Gew.-% Bariumchromat

**Cu 0865 T 3/16***
55.0-65.0 Gew.-% Kupferoxid
25.0-35.0 Gew.-% Calciumsilikat
5.0-10.0 Gew.-% Palygorskit ($[Mg(Al_{0.5}$-$1Fe_{0-0.5})]Si_4(OH)O_{10.4}H_2O$)
1.0-5.0 Gew.-% Graphit
1.0-5.0 Gew.-% Siliciumdioxid
0.5-1.5 Gew.-% Silica (kristallin).

*alle Angaben bezogen auf das Gesamtgewicht des trockenen Katalysators.

6. Verfahren nach einer der vorhergehenden Ausführungsformen, wobei der Katalysator als homogener oder heterogener Katalysator eingesetzt wird. Insbesondere die heterogene Katalyse der Umsetzung von Isopulegol im Festbettreaktor stellt eine bevorzugte Ausführungsform der Erfindung dar.

7. Verfahren nach einer der vorhergehenden Ausführungsformen, wobei der Katalysator vor der Umsetzung mit Wasserstoff oder Wasserstoff und einem Alkohol (gleichzeitig oder zeitlich versetzt in beliebiger Reihenfolge), gegebenenfalls in einem Trägergasstrom, aktiviert wird.

Die Temperatur für die Aktivierung kann zwischen 150 und 220 °C liegen.

Eine bevorzugte Ausführungsform des Verfahrens ist die Aktivierung des Katalysators zunächst durch einen Wasserstoffstrom, der insbesondere unterstützt durch einen Trägergasstrom durch den Reaktor strömt. In einem zeitlich versetzten Verfahrensschritt erfolgt in dieser bevorzugten Ausführungsform nach der reduktiven Aktivierung die Aktivierung des Katalysators durch einen Alkohol, der mit oder ohne, insbesondere ohne Trägergasunterstützung durch den Reaktor geführt wird.

Eine weitere bevorzugte Ausführungsform des Verfahrens umfasst die zeitlich versetzte Aktivierung des Katalysators, zunächst durch Wasserstoff, danach durch einen Alkohol, wobei in keinem der Aktivierungsschritte Trägergas zugesetzt wird.

Eine weitere bevorzugte Ausführungsform des Verfahrens umfasst die Aktivierung des Katalysators zunächst durch Wasserstoff, danach durch Alkohol, insbesondere unter Zusatz von Trägergas in beiden Aktivierungsschritten.

Weitere spezielle Ausgestaltungen des erfindungsgemäßen Verfahrens umfassen die zeitlich versetzte Aktivierung des Katalysators zuerst durch einen Alkohol, danach durch Wasserstoff. Beispielsweise kann in einem, in beiden oder in keinem der Aktivierungsschritte Trägergas zugesetzt werden.

Ferner ist die gleichzeitige Aktivierung des Katalysators mit Wasserstoff und Alkohol eine weitere Ausführungsform des erfindungsgemäßen Verfahrens. Dabei kann die Aktivierung beispielsweise unterstützt durch Trägergasstrom oder ohne Trägergasstrom durchgeführt werden.

Die wie oben beschrieben aktivierten Katalysatoren können unter geeigneten Lösungsmitteln, insbesondere aliphatischen linearen oder verzweigten Alkoholen, vorzugsweise Isononanol, aufbewahrt werden.

8. Verfahren nach einer der vorhergehenden Ausführungsformen, wobei als Trägergas Stickstoff oder Argon oder Gemische davon verwendet werden. Dabei ist insbesondere die Verwendung von Stickstoff eine bevorzugte Ausführungsform des Verfahrens. Insbesondere das Trägergas-basierte Durchströmen des Reaktors während der erfindungsgemäßen Umsetzung und während der Aktivierung des Katalysators stellt eine bevorzugte Ausführungsform

des erfindungsgemäßen Verfahrens dar. Weiterhin kann der Trägergasstrom während der Umsetzung bis zu 10 Vol.-% , wie z.B. 0,1 bis 10 Vol.-% Wasserstoff enthalten.

9. Verfahren nach Ausführungsform 8, wobei der Reaktor mit einer Rate von 0 bis 5 NL/h Trägergas pro g/h Substrat, insbesondere 0,5 bis 1,5 NL/h Trägergas pro g/h Substrat, durchströmt wird.

10. Verfahren nach einer der vorhergehenden Ausführungsformen, wobei der für die Aktivierung verwendete Alkohol ausgewählt ist unter gesättigten oder ein- oder mehrfach ungesättigten, geradkettigen oder verzweigten oder cyclischen aliphatischen oder aromatischen, insbesondere gesättigten oder ein- oder mehrfach ungesättigten, geradkettigen oder verzweigten aliphatischen, Mono- oder Polyolen, insbesondere Mono-oder Diolen, oder Kombinationen davon.

11. Verfahren nach einer der vorhergehenden Ausführungsformen, wobei der für die Aktivierung verwendete Alkohol, insbesondere Alkanol oder Alkenol, mindestens eine primäre und/oder mindestens eine sekundäre OH-Gruppe umfasst.

Darunter fallen beispielsweise Alkohole, insbesondere Alkanole oder Alkenole, die eine, zwei oder drei primäre OH-Gruppen, vorzugsweise eine primäre OH-Gruppe, aufweisen. Weiterhin fallen darunter Alkohole, insbesondere Alkanole oder Alkenole, mit einer primären und einer sekundären OH-Gruppe, Alkohole, insbesondere Alkanole oder Alkenole, mit zwei primären OH-Gruppen und einer sekundären OH-Gruppe, Alkohole, insbesondere Alkanole oder Alkenole, mit einer primären OH-Gruppe und zwei sekundären OH-Gruppen und Alkohole, insbesondere Alkanole oder Alkenole, mit einer, zwei oder drei sekundären OH-Gruppen.

Bevorzugt sind Alkohole, insbesondere Alkanole oder Alkenole, mit einer primären OH-Gruppe, zwei primären OH-Gruppen oder einer primären und einer sekundären OH-Gruppe.

Insbesondere die Verwendung von Alkoholen mit gesättigten oder ein- oder mehrfach ungesättigten, geradkettigen oder verzweigten aliphatischen Kohlenwasserstoffresten, mit 1 bis 20 Kohlenstoffatomen Kohlenstoffatomen stellt eine bevorzugte Ausführungsform der Erfindung dar. Insbesondere sind zu nennen aliphatische $C_1$-$C_{20}$-, $C_3$-$C_{16}$- oder $C_4$-$C_{12}$ Reste. Darunter sind insbesondere Alkohole, insbesondere Alkanole oder Alkenole, bei denen unter den Reaktionsbedingungen nicht mit einer Kondensation zu rechnen ist, z.B. 1,4-Butandiol, Hexanol, Cyclohexanol, Octanol, 1-Nonanol und Citronellol, eine bevorzugte Ausführungsform der Erfindung.

Unter die genannten Alkohole fallen beispielsweise Monoole oder Diole mit drei Kohlenstoffatomen, z.B. Propan-1-ol, Propan-2-ol, Propan-1,2-diol und Propan-1,3-diol, sowie Mono- oder Diole mit vier Kohlenstoffatomen, wie z.B. Butan-1-ol, Butan-2-ol, 2-Methylpropan-1-ol, Butan-1,2-diol, Butan-1,3-diol, Butan-1,4-diol, Butan-2,3-diol, 2-Methylpropan-1,3-diol und 2-Methylpropan-1,2-diol.

Unter die genannten Alkohole fallen auch Pentanole mit mindestens einer primären oder einer sekundären OH-Gruppe, wie beispielsweise die Monoole Pentan-1-ol, Pentan-2-ol, Pentan-3-ol, 2-Methylbutan-1-ol, 2-Methylbutan-2-ol, 3-Methylbutan-1ol, 3-Methylbutan-2-ol, 2,2-Dimethylpropan-1-ol und die Diole Pentan-1,2-diol, Pentan-1,3-diol, Pentan-1,4-diol, Pentan-1,5-diol, Pentan-2,3-diol, Pentan-2,4-diol, Pentan-2,5-diol, sowie die verzweigten Strukturisomere davon.

Unter "Hexanol" fallen vorzugsweise folgende Monoole mit mindestens einer primären oder einer sekundären OH-Gruppe: Hexan-1-ol, Hexan-2-ol, Hexan-3-ol, 2-Methylpentan-1-ol, 3-Methylpentan-1-ol, 4-Methylpentan-1-ol, 3-Methylpentan-2-ol, 4-Methylpentan-2-ol, 2-Methylpentan-3-ol, 2,2-Dimethylbutan-1-ol, 2,3-Dimethylbutan-1-ol, 3,3-Dimethylbutan-1-ol, 3,3-Dimethylbutan-2-ol und 2-Ethylbutan-1-ol. Weiterhin fallen unter "Hexanol" die Diole 1,2-Hexandiol, 1,3-Hexandiol, 1,4-Hexandiol, 1,5-Hexandiol, 1,6-Hexandiol, 2,3-Hexandiol, 2,4-Hexandiol, 2,5-Hexandiol und 3,4-Hexandiol, sowie die verzweigten Strukturisomere davon.

Unter die genannten Alkohole fallen auch Heptanole mit mindestens einer primären oder einer sekundären OH-Gruppe, wie beispielsweise die Monoole Heptan-1-ol, Heptan-2-ol, Heptan-3-ol, Heptan-4-ol, 2-Methylhexan-1-ol, 2-Methylhexan-3-ol, 2-Methylhexan-4-ol, 2-Methylhexan-5-ol, 2-Methylhexan-6-ol, 3-Methylhexan-1-ol, 3-Methylhexan-2-ol, 3-Methylhexan-4-ol, 3-Methylhexan-5-ol, 3-Methylhexan-6-ol, 2,2-Dimethylpentan-1-ol, 2,2-Dimethylpentan-3-ol, 2,2-Dimethylpentan-4-ol, 2,2-Dimethylpentan-5-ol, 2,3-Dimethylpentan-1-ol, 2,3-Dimethylpentan-4-ol, 2,3-Dimethylpentan-5-ol, 2,4-Dimethylpentan-1-ol, 2,4-Dimethylpentan-3-ol, 3,3-Dimethylpentan-1-ol, 3,3-Dimethylpentan-2-ol, 3-Ethylpentan-1-ol, 3-Ethylpentan-2-ol, 2,2,3-Trimethylbutan-1-ol, 2,2,3-Trimethylbutan-4-ol, und die Diole Heptan-1,2-diol, Heptan-1,3-diol, Heptan-1,4-diol, Heptan-1,5-diol, Heptan-1,6-diol, Heptan-1,7-diol, Heptan-2,3-diol, Heptan-2,4-diol, Heptan-2,5-diol, Heptan-2,6-diol, Heptan-2,7-diol, Heptan-3,4-diol, Heptan-3,5-diol, sowie die verzweigten Strukturisomere davon.

Unter "Octanol" fallen vorzugsweise folgende Monoole mit einer primären oder einer sekundären OH-Gruppe: Octan-1-ol, Octan-2-ol, Octan-3-ol, Octan-4-ol, 2-Methylheptan-1-ol, 2-Methylheptan-3-ol, 2-Methylheptan-4-ol, 2-Methylheptan-5-ol, 2-Methylheptan-6-ol, 2-Methylheptan-7-ol, 3-Methylheptan-1-ol, 3-Methylheptan-2-ol, 3-Methylheptan-4-ol, 3-Methylheptan-5-ol, 3-Methylheptan-6-ol, 3-Methylheptan-7-ol, 4-Methylheptan-1-ol, 4-Methylheptan-2-ol, 4-Methylheptan-3-ol, 4-Methylheptan-5-ol, 4-Methylheptan-6-ol, 4-Methylheptan-7-ol, 2,2-Dimethylhexan-1-ol, 2,2-Dimethylhexan-3-ol, 2,2-Dimethylhexan-4-ol, 2,2-Dimethylhexan-5-ol, 2,2-Dimethylhexan-6-ol, 2,3-Dimethylhexan-1-ol, 2,3-Dimethylhexan-4-ol, 2,3-Dimethylhexan-5-ol, 2,3-Dimethylhexan-6-ol, 2,4-Dimethylhexan-1-ol, 2,4-Dimethylhexan-3-ol, 2,4-Dimethylhexan-5-ol, 2,4-Dimethylhexan-6-ol, 2,5-Dimethylhexan-1-ol, 2,5-Dimethylhexan-3-ol, 2,5-Dimethylhexan-4-ol, 2,5-Dimethylhexan-6-ol, 3,3-Dimethylhexan-1-ol, 3,3-Dimethylhexan-2-ol, 3,3-Dimethylhexan-4-ol, 3,3-Dimethylhexan-5-ol, 3,3-Dimethylhexan-6-ol, 3,4-Dimethylhexan-1-ol, 3,4-Dimethylhexan-2-ol, 3-Ethylhexan-1-ol, 3-Ethylhexan-2-ol, 3-Ethylhexan-4-ol, 3-Ethylhexan-5-ol, 3-Ethylhexan-6-ol, 2,2,3-Trimethylpentan-1-ol, 2,2,3-Trimethylpentan-4-ol, 2,2,3-Trimethylpentan-5-ol, 2,2,4-Trimethylpentan-1-ol, 2,2,4-Trimethylpentan-3-ol, 2,2,4-Trimethylpentan-5-ol, 2,3,3-Trimethylpentan-1-ol, 2,3,3-Trimethylpentan-4-ol, 2,3,3-Trimethylpentan-5-ol, 2,3,4-Trimethylpentan-1-ol, 3-Ethyl-2-methylpentan-1-ol, 3-Ethyl-2-methylpentan-4-ol, 3-Ethyl-2-methylpentan-5-ol, 3-Ethyl-3-methylpentan-1-ol, 3-Ethyl-3-methylpentan-2-ol, 3-Ethyl-3-methylpentan-4-ol, 2,2,3,3-Tetramethylbutan-1-ol. Unter "Octanol" fallen zudem die Diole 1,2-Octandiol, 1,3-Octandiol, 1,4-Octandiol, 1,5-Octandiol, 1,6-Octandiol, 1,7-Octandiol, 1,8-Octandiol, 2,3-Octandiol, 2,4-Octandiol, 2,5-Octandiol, 2,6-Octandiol, 3,4-Octandiol, 3,5-Octandiol, 3,6-Octandiol, 4,5-Octandiol, sowie die verzweigten Strukturisomere davon.

Weiterhin stellen Alkohole, insbesondere Alkanole oder Alkenole, deren Verwendung die Bewahrung der Konfiguration des Stereozentrums an Position 5 von Isopulegol begünstigt, wie insbesondere 1-Nonanol, 1,4-Butandiol und Citronellol, eine bevorzugte Ausführungsform der Erfindung dar.

Ganz besonders bevorzugt ist die Verwendung von Alkoholen, insbesondere Alkanolen oder Alkenolen mit einer primären OH-Gruppe, z.B. Citronellol, 1-Nonanol oder Mischungen davon oder von Alkoholen, insbesondere Alkanolen oder Alkenolen mit zwei primären OH-Gruppen, z.B. 1,4-Butandiol.

12. Verfahren nach einer der vorhergehenden Ausführungsformen, wobei die Umsetzung von Isopulegol in einer Gasphasenapparatur mit gekoppeltem Verdampfer durchgeführt wird. Dabei ist der Verdampfer beispielsweise oberhalb des Reaktors angebracht und dient dazu, die flüssigen Reaktionskomponenten, insbesondere Isopulegol und den gegebenenfalls zur Aktivierung verwendeten Alkohol, zu verdampfen und die eingesetzten Gasströme, z.B. Wasserstoff, Stickstoff oder Argon, gegebenenfalls, falls nicht bereits vorgeheizt, aufzuheizen.

13. Verfahren nach einer der vorhergehenden Ausführungsformen, wobei das Umsetzungsprodukt Menthon (2) und/oder Isomenthon (3) umfasst.

In einer bevorzugten Ausführungsform der Erfindung sind Menthon und Isomenthon die Hauptprodukte, als Nebenprodukte fallen beispielsweise Menthol (1) und Thymol (7) an. Insbesondere die Umsetzung zu Menthon und Isomenthon mit (R)-Konfiguration des Stereozentrums in Position 5 ist eine bevorzugte Ausführungsform des erfindungsgemäßen Verfahrens.

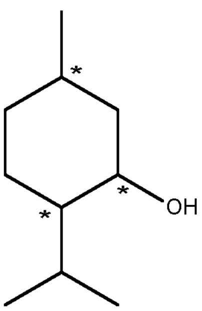

Menthol (1)

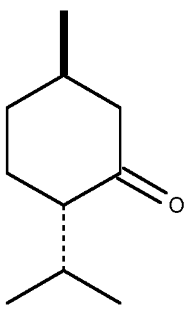

Menthon (2)

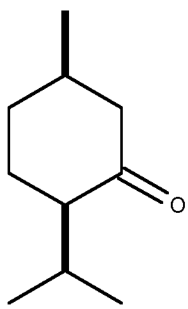

Isomenthon (3)

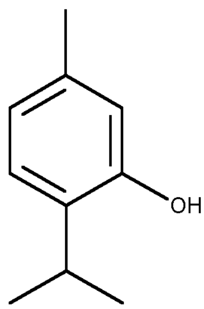

Thymol (7)

Insbesondere bevorzugt ist ein Verfahren, wobei das Umsetzungsprodukt Menthon und/oder Isomenthon mit einem erhöhten Anteil, vorzugsweise mehr als 70%, insbesondere mehr als 80%, ganz besonders bevorzugt mehr als 90%, z.B. wenigstens 95%, 96%, 97%, 98%, 99% oder 99,9% des Enantiomers mit (R)-Konfiguration in Position 5 umfasst.

14. Verfahren nach einer der vorhergehenden Ausführungsformen, wobei im Umsetzungsprodukt Menthon (8) und/oder Isomenthon (9) im Wesentlichen enantiomerenrein vorliegt.

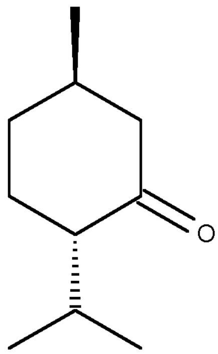

Menthon (8)

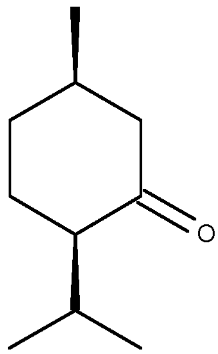

Isomenthon (9)

15. Verfahren zur Herstellung eines Produktes ausgewählt unter Lebensmitteln, Kosmetika, pharmazeutischen Erzeugnissen, Tabakformulierungen, Haushaltsprodukten und Wäschepflegeprodukten, wobei man einen Menthon-haltigen Zusatz nach einem der vorhergehenden Ausführungsformen herstellt und diesen Zusatz dem Produkt zugibt.

**c) Detaillierte Beschreibung des erfindungsgemäßen Verfahrens**

Anhand einer bevorzugten Ausführungsform zur Umsetzung von Isopulegol wird das erfindungsgemäße Verfahrensprinzip im Folgenden näher erläutert.

Die Gasphasenapparatur umfasst einen Reaktor, der beispielsweise ein oder mehrere Rohre beinhaltet, die entweder elektrisch, mit Wärmeträgerflüssigkeit oder mit heißen Gasen (z.B. Rauchgas) beheizt werden können. Der Festbettkatalysator liegt im Reaktor, beispielsweise in eines der Rohre gefüllt, vor. Im Katalysatorbett kann die Reaktionstemperatur gemessen werden. Das mit Substrat beladene Trägergas durchströmt das Katalysatorbett, zum Beispiel von oben nach unten. Gekoppelt an den Reaktor befindet sich ein Verdampfer, der beispielsweise oberhalb des Reaktors angebracht ist und durch dessen Verwendung die (flüssigen) Reaktanden in die Gasphase überführt werden können, ggf. unterstützt durch Einsatz von Trägergas. Auch der Trägergasstrom kann dann mittels des Verdampfers oder mittels eines separaten Vorheizers auf die Reaktionstemperatur aufgeheizt werden. Die Reaktionsprodukte kondensieren nach Austritt aus dem Reaktor entweder spontan, durch Quench mit kaltem Gas oder Flüssigkeit oder durch Kondensation an gekühlten Flächen. Der Übergang zwischen Verdampfer und Reaktor ist zum Zwecke der Vermeidung von Kondensation gut isoliert oder gar schutzbeheizt (zum Beispiel mit einem Heizband umwickelt).

In einer speziellen Ausführungsform wird der Gasphasenreaktor beispielsweise mit einem Kupferkatalysator, z.B. X540T 1/8, gefüllt und der Katalysator unter $H_2$-haltigem Gasstrom aktiviert. Die Temperatur für die Aktivierung kann zwischen 150 und 220 °C liegen. Verdampfer und Reaktor werden beispielsweise bei einer Temperatur von 150-220 °C bei 50 mBar bis 1000 mBar oder bis 1 Bar Überdruck betrieben. Bevorzugte Vakuumfahrweise sind 500 mBar, noch bevorzugter ist die Fahrweise bei Normaldruck, und unter Verwendung von Trägergas, wie zum Beispiel Stickstoff oder Argon und gegebenenfalls in Gegenwart von bis zu 10 Vol.-% Wasserstoff. Isopulegol wird kontinuierlich in den Verdampfer eingebracht. Das Produktgemisch wird am Reaktorausgang kondensiert und die Zusammensetzung gaschromatographisch analysiert.

Nach Umsetzung (z.B. nach 1 bis 24, 1 bis 20, 2 bis 11 oder etwa 5 Stunden) werden Reaktor und Verdampfer unter Trägergasstrom abgekühlt und die Umsetzung, z.B. am Folgetag, ohne Katalysatorwechsel für einen oder mehrere Reaktionszyklen fortgesetzt. Alternativ wird die Umsetzung kontinuierlich ohne Unterbrechung durchgeführt. Nach jeweils definierten Reaktionszeiten, zum Beispiel eine Stunde, werden Umsatz, Massenbilanz und Produktzusammensetzung über Gaschromatographie bestimmt. Die detektierten Produkte umfassen beispielsweise Menthon, Isomenthon, Thymol und Menthol.

Wird bei der Ausführung des erfindungsgemäßen Verfahrens beispielsweise enantiomerenangereichertes Isopulegol als Ausgangsmaterial verwendet, kann weiterhin ein Menthon-/Isomenthongemisch in enantiomerenangereicherter Form detektiert werden. Der Enantiomerenüberschuss kann über chirale Gaschromatographie bestimmt werden. Dabei ist für das Erreichen eines hohen Enantiomerenüberschusses insbesondere bevorzugt das Katalysatorsystem nach der ersten reduktiven Aktivierung durch Wasserstoff noch ein zweites Mal durch die Verwendung eines Alkohols, beispielsweise 1-Nonanol-, 1,4-Butandiol- oder Citronellol, zu aktivieren.

Bei der Verwendung von Citronellol können z.B. Enantiomerenüberschüsse größer als 99% erreicht werden, für 1-Nonanol Werte von bis zu 98.4% und für 1,4-Butandiol Werte von bis zu 93.4%. In den genannten Umsetzungen ist beispielsweise das Enantiomer mit (R)-Konfiguration in Position 5 das bevorzugte.

Die Erfindung wird nun unter Bezugnahme auf folgende Beispiele näher erläutert.

## Experimenteller Teil

### A) Allgemeine Arbeitsmethoden

Die nachfolgenden Umsetzungen wurden in einer Gasphasenapparatur, umfassend einen Reaktor mit doppelwandigem Glasrohr, dessen inneres Rohr elektrisch beheizt werden kann und am unteren Ende eine Lochplatte enthält, durchgeführt. Der Reaktor wird mit dem Katalysator befüllt, dessen Aktivierung reduktiv durch einen $H_2$-haltigen Gasstrom (20-40 NL/h) bei einer Temperatur von 170-180 °C durchgeführt wird. Nach der Aktivierung des Katalysators wird der Reaktor unter Inertgasatmosphäre betrieben, das Trägergas Stickstoff strömt von oben nach unten durch den Reaktor. Die flüssigen Ausgangsmaterialien werden unter Verwendung eines Verdampfers, der analog dem Reaktor aufgebaut ist und oberhalb des Reaktors angebracht ist, in die Gasphase überführt. Die Kondensation des Produktgemisches erfolgt durch Wasserkühlung.

Die angegebenen Selektivitäten, Umsätze und Massenbilanzen wurden gaschromatographisch bestimmt und unter Verwendung eines Agilent 7890 A Gaschromatographen durchgeführt. Enantiomerenüberschüsse wurden mittels chiraler Gaschromatographie unter Verwendung eines Agilent 7890 A Gaschromatographen ermittelt.

**Gaschromatographie**

| | |
|---|---|
| Gerät | : Agilent 7890 A |
| Säule | : 50 m CP-Wax, Innendurchmesser 0,32 mm, Filmdicke 1,2 μm |
| Eluent | : Stickstoff |
| Detektor | : FID, 250 °C |
| Injektion | : 0,2 μL, Split 100:1 |
| Temperatur | : Start 130 °C, 3 °C/min auf 150 °C, 20 min isotherm bei 150 °C, 10°C/min auf 240°C |
| Laufzeit | : 60 min |
| Druck | : 46,671 kPa (Druck geregelt) |

**Chirale Gaschromatographie**

| | |
|---|---|
| Gerät | : Agilent 7890 A |
| Säule | : 30 m BGB-174 S, Innendurchmesser 0,25 mm, Filmdicke 0,25 μm |
| Eluent | : Helium |
| Detektor | : FID, 250 °C |
| Flussrate | : 1,5 mL/min (Fluss geregelt) |
| Injektion | : 0,2 μL, Split 100:1 |
| Temperatur | : Start 60 °C, 2 °C/min auf 80 °C, 30 min isotherm bei 80 °C, 10 °C/min auf 210 °C |
| Laufzeit | : 53 min |

### B) Herstellungbeispiele

**Beispiel 1: Umsetzung von Isopulegol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt.

Der Umsatz von Isopulegol war über die gesamte Reaktionsdauer vollständig.

**Tabelle 1: Umsatz, Massenbilanz und Produktzusammensetzung bei der Umsetzung von Isopule-gol.**

| Versuch | Zeit [h] | Umsatz Isopulegol [%]* | Selektivität Menthon [%]* | Selektivität Isomenthon [%]* | Selektivität Menthone (gesamt) [%]* | Selektivität Thymol [%]* | Massenbilanz [%] |
|---|---|---|---|---|---|---|---|
| 1 | 5 | 100 | 50,4 | 25,6 | 76,0 | 15,4 | 95 |
| 2 | 5 | 100 | 56,6 | 28,0 | 84,6 | 8,5 | > 98 |
| 3 | 5 | 100 | 59,2 | 29,3 | 88,5 | 6,3 | 98 |
| *Angaben basierend auf Flächenprozent. | | | | | | | |

Der Umsatz von Isopulegol zu Menthon erfolgt in guten bis sehr guten Ausbeuten bis zu 88,5%. Das Verhältnis von Menthon zu Isomenthon liegt für alle Versuche im Bereich 65/35 bis 70/30 (Menthon/Isomenthon) (siehe Tabelle 1).

**Beispiel 2: Umsetzung von enantiomerenangereichertem Isopulegol (ee > 99%); Aktivierung des Katalysators mit Citronellol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20-40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (*ee* > 99%, Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt. Zwischen Versuch 6 und 7 wurde der Reaktor für fünf Stunden bei 170 °C mit Citronellol (15 g/h, 96.0 mmol/h) durchströmt.

**Tabelle 2: Übersicht über die Produktzusammensetzung bei der Umsetzung von Isopulegol (ee > 99%).**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%]* | ee [%] | Selektivität [%]* | ee [%] | | | |
| 1 | 5 | 100 | 50,3 | 65,8 | 25,1 | 67,4 | 75,4 | 14,3 | 95 |
| 2 | 5 | 100 | 55,0 | 75,5 | 26,3 | 77,8 | 81,3 | 9,8 | > 98 |
| 3 | 5 | 100 | 58,4 | 84,7 | 28,1 | 85,8 | 86,5 | 7,1 | > 98 |
| 4 | 5 | 100 | 58,5 | 85,1 | 28,7 | 86,3 | 87,2 | 6,7 | 98 |
| 5 | 5 | 99,5 | 58,3 | 85,8 | 29,1 | 87,4 | 87,4 | 6,7 | > 98 |
| 6 | 5 | 97,7 | 58,3 | 89,2 | 29,7 | 90,2 | 88,0 | 6,8 | > 98 |
| 7 | 5 | 51,5 | 42,9 | > 99 | 24,5 | > 99 | 67,4 | 0,8 | > 98 |
| 8 | 5 | 35,2 | 34,4 | > 99 | 19,9 | > 99 | 54,3 | 0,3 | > 98 |

*Angaben basierend auf Flächenprozent.

Die Umsätze von Isopulegol zu Menthon (gesamt) steigen bis einschließlich Versuch 6 sukzessive bis auf einen Wert von 88% an. Ähnlich verhält es sich mit den Enatiomerenüberschüssen: Ausgehend von mäßigen Enantiomerenüberschüssen für Versuch 1 ist in der Folge eine Steigerung auf bis zu 90% ee bei Versuch 6 zu beobachten. Nach der Aktivierung des Katalysators durch Citronellol geht der Umsatz zwar deutlich zurück (bis zu 65% an Ausgangsmaterial werden nicht umgesetzt). Der Enantiomerenüberschuss jedoch wird in den Versuchen 7 und 8 überraschenderweise auf größer als 99% gesteigert (siehe Tabelle 2).

**Beispiel 3: Umsetzung von enantiomerenangereichertem Isopulegol (ee > 99%); Aktivierung des Katalysators mit Citronellol**

Der Gasphasenreaktor wurde mit X540T 1/8 (150 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-Gasstrom (20 - 40 NL/h) bei einer Temperatur von 170-180 °C aktiviert. Im Folgenden wurden Verdampfer und Reaktor bei einer Temperatur von 170 °C betrieben und bei Normaldruck von Stickstoff (20 NL/h) durchströmt. Isopulegol (*ee* > 99%, Wassergehalt 3.7 Gew.-%, 15 g/h, 97.2 mmol/h) wurde kontinuierlich in den Verdampfer eingebracht. Das Produktgemisch wurde am Reaktorausgang kondensiert und die Zusammensetzung gaschromatographisch analysiert. Nach jeweils fünf Stunden Versuchsdauer wurden Reaktor und Verdampfer unter Stickstoffstrom (20 NL/h) abgekühlt und der Versuch nach 18 h ohne Katalysatorwechsel fortgesetzt. Zwischen Versuch 1 und 2 wurde der Reaktor für fünf Stunden bei 170 °C mit Citronellol (15 g/h, 96.0 mmol/h) durchströmt. Vor Versuch 11 erfolgte eine Temperaturerhöhung auf 180 °C.

**Tabelle 3: Übersicht über die Produktzusammensetzung bei der Umsetzung von Isopulegol (ee > 99%).**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%]* | ee [%] | Selektivität [%]* | ee [%] | | | |
| 1 | 5 | 100 | 52,8 | 71,0 | 25,8 | 71,3 | 78,6 | 12,4 | 94 |
| 2 | 5 | 92,8 | 58,3 | 98,3 | 30,1 | 98,6 | 88,4 | 2,4 | > 98 |
| 3 | 5 | 92,3 | 58,7 | 98,3 | 30,6 | 98,6 | 89,3 | 2,4 | > 98 |
| 4 | 5 | 90,0 | 58,4 | 98,5 | 30,6 | 98,4 | 89,0 | 2,4 | > 98 |
| 5 | 5 | 89,4 | 58,4 | 98,3 | 30,7 | >99 | 89,1 | 2,3 | > 98 |
| 6 | 5 | 87,6 | 57,9 | 98,5 | 30,4 | 98,6 | 88,3 | 2,0 | > 98 |
| 7 | 5 | 86,6 | 57,7 | 98,6 | 30,3 | 98,7 | 88,0 | 2,0 | > 98 |
| 8 | 5 | 84,1 | 57,1 | 98,8 | 30,0 | > 99 | 87,1 | 1,7 | > 98 |
| 9 | 5 | 82,6 | 56,5 | 98,9 | 29,7 | > 99 | 86,2 | 1,6 | > 98 |
| 10 | 5 | 78,8 | 55,5 | 99,0 | 29,2 | > 99 | 84,7 | 1,4 | > 98 |
| 11** | 5 | 89,0 | 57,1 | 98,2 | 30,2 | > 99 | 87,3 | 2,3 | > 98 |
| 12** | 5 | 87,7 | 56,6 | 98,3 | 30,0 | 98,6 | 86,6 | 2,2 | > 98 |

*Angaben basierend auf Flächenprozent; **Reaktionstemperatur 180 °C

Durch Aktivierung des Katalysators mit Citronellol konnten auch in diesem Fall die Enantiomerenüberschüsse von Versuch 2 an auf größer als 98% erhöht werden. Der Umsatzrückgang auf unter 80% in Versuch 10 konnte überraschenderweise durch eine mäßige Temperaturerhöhung um 10 °C kompensiert werden. Hohe Enantiomerenüberschüsse werden also nicht zwingend auf Kosten des Umsatzes erreicht. (siehe Tabelle 3).

**Beispiel 4: Umsetzung von enantiomerenangereichertem Isopulegol (ee > 99%); Aktivierung des Katalysators mit 1-Nonanol**

Der Gasphasenreaktor wurde mit X540T 1/8 (75 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 1-Nonanol (7.5 g/h, 52.0 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (*ee* > 99%, Wassergehalt 3.7 Gew.-%, 7.5 g/h, 48.6 mmol/h) wurde kontinuierlich in den Verdampfer eingebracht. Das Produktgemisch wurde am Reaktorausgang kondensiert und die Zusammensetzung gaschromatographisch analysiert.

**Tabelle 4: Übersicht über die Produktzusammensetzung bei der Umsetzung von Isopulegol (ee > 99%) bei Aktivierung des Katalysators mit 1-Nonanol.**

| Ver-such | ZeitZeit [h] | Umsatz Isopulegol [%] * | Menthon | | Isomenthon | | Selek-tivität Menthone (gesamt) [%]* | Selek-tivität Thymol [%]* | Massenbilanz [%] |
|---|---|---|---|---|---|---|---|---|---|
| | | | Selektivität [%]* | ee [%] | Selektivität [%]* | ee [%] | | | |
| 1 | 5 | 96,8 | 56,8 | 98,2 | 32,1 | 98,4 | 88,9 | 3,3 | > 98 |

*Angaben basierend auf Flächenprozent.

Die Behandlung des mit Wasserstoff aktivierten Katalysators mit 1-Nonanol führt bei hohen Umsätzen zu Menthon in enatiomerenangereicherter Form. Überraschenderweise können auch für die Verwendung dieses Alkohols Enantiomerenüberschüsse von über 98% erreicht werden (siehe Tabelle 4).

**Beispiel 5: Umsetzung von enantiomerenangereichertem Isopulegol (ee > 99%); Aktivierung des Katalysators mit 1,4-Butandiol**

Der Gasphasenreaktor wurde mit X540T 1/8 (75 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 1,4-Butandiol (7.5 g/h, 83.2 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (*ee* > 99%, Wassergehalt 3.7 Gew.-%, 7.5 g/h, 48.6 mmol/h) wurde kontinuierlich in den Verdampfer eingebracht. Das Produktgemisch wurde am Reaktorausgang kondensiert und die Zusammensetzung über gaschromatographische Untersuchungen analysiert.

**Tabelle 5: Übersicht über die Produktzusammensetzung bei der Umsetzung von Isopulegol (ee > 99%) bei Aktivierung des Katalysators mit 1,4-Butandiol.**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%]* | ee [%] | Selektivität [%]* | ee [%] | | | |
| 1 | 5 | 96,1 | 56,5 | 93,0 | 28,8 | 93,4 | 85,3 | 1,8 | > 98 |
| *Angaben basierend auf Flächenprozent. | | | | | | | | | |

Die Behandlung des mit Wasserstoff aktivierten Katalysators mit 1,4-Butandiol führt ebenfalls zu guten Umsätzen von Isopulegol zu Menthon. Die Enantiomerenüberschüsse bleiben jedoch geringfügig hinter den für 1-Nonanol und Citronellol erreichten Ergebnissen zurück. Die Eignung von Diolen für diese Umsetzung konnte jedoch erfolgreich demonstriert werden (siehe Tabelle 5).

**Beispiel 6: Umsetzung von enantiomerenangereichertem Isopulegol (ee > 99%); Aktivierung des Katalysators mit 3-Pentanol**

Der Gasphasenreaktor wurde mit X540T 1/8 (75 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 3-Pentanol (7.5 g/h, 85.1 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (*ee* > 99%, Wassergehalt 3.7 Gew.-%, 7.5 g/h, 48.6 mmol/h) wurde kontinuierlich in den Verdampfer eingebracht. Das Produktgemisch wurde am Reaktorausgang kondensiert und die Zusammensetzung gaschromatographisch analysiert.

**Tabelle 6: Übersicht über die Produktzusammensetzung bei der Umsetzung von Isopulegol (*ee* > 99%) bei Aktivierung des Katalysators mit 3-Pentanol.**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%] * | *ee* [%] | Selektivität [%] * | *ee* [%] | | | |
| 1 | 5 | 100 | 48,5 | 70,2 | 24,6 | 71,6 | 73,1 | 10,7 | 89 |
| 2 | 24 | 100 | 52,3 | 80,5 | 27,0 | 81,5 | 79,3 | 8,3 | 93 |
| 3 | 24 | 93,4 | 54,7 | 91,1 | 30,6 | 92,6 | 85,3 | 4,6 | 95 |
| 4 | 4 | 88,9 | 52,6 | 94,9 | 30,8 | 96,0 | 83,4 | 2,9 | 96 |

*Angaben basierend auf Flächenprozent.

Nach Behandlung des Katalysators mit 3-Pentanol kann Isopulegol bei hohem Umsatz und guter Selektivität zu Menthon/Isomenthon umgesetzt werden. Der Enantiomerenüberschuss liegt nicht sofort bei > 90 %, sondern steigt von anfangs ca. 70 % im Verlauf von 52 Stunden auf ca. 95 % an.

**Beispiel 7: Umsetzung von enantiomerenangereichertem Isopulegol (ee > 99%); Aktivierung des Katalysators mit Isopropanol**

Der Gasphasenreaktor wurde mit X540T 1/8 (50 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von Isopropanol (5 g/h, 83.2 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (*ee* > 99%, Wassergehalt 3.7 Gew.-%, 5 g/h, 32.4 mmol/h) wurde kontinuierlich in den Verdampfer eingebracht. Das Produktgemisch wurde am Reaktorausgang kondensiert und die Zusammensetzung gaschromatographisch analysiert.

**Tabelle 7: Übersicht über die Produktzusammensetzung bei der Umsetzung von Isopulegol (*ee* > 99%) bei Aktivierung des Katalysators mit Isopropanol.**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%]* | *ee* [%] | Selektivität [%]* | *ee* [%] | | | |
| 1 | 5 | 99,2 | 51,9 | 81,9 | 26,8 | 83,9 | 78,7 | 7,8 | 89 |

*Angaben basierend auf Flächenprozent.

Nach Behandlung mit Isopropanol wurde Menthon aus Isopulegol bei hohem Umsatz und hoher Selektivität erhalten. Der Enantiomerenüberschuss lag ab der dritten Versuchsstunde konstant bei knapp über 80 %.

**Beispiel 8: Umsetzung von enantiomerenangereichertem Isopulegol (ee > 99%); Aktivierung des Katalysators mit 1,3-Butandiol**

Der Gasphasenreaktor wurde mit X540T 1/8 (50 g, 30-40% Kupferoxid, 10-25% Aluminiumoxid, 10-25% Manganoxid und 30-40% Aluminiumkupferoxid ($Al_2CuO_4$)) gefüllt und der Katalysator unter $H_2$-haltigem Gasstrom (20 - 40 NL/h) bei einer Temperatur von 170-180 °C aktiviert. Anschließend wurde der Reaktor von 1,3-Butandiol (5 g/h, 55.5 mmol/h) durchströmt. Verdampfer und Reaktor wurden im Folgenden bei einer Temperatur von 170 °C betrieben und bei Normaldruck von Stickstoff (20 NL/h) durchströmt. Isopulegol (*ee* > 99%, Wassergehalt 3.7 Gew.-%, 5 g/h, 32.4 mmol/h) wurde kontinuierlich in den Verdampfer eingebracht. Das Produktgemisch wurde am Reaktorausgang kondensiert und die Zusammensetzung gaschromatographisch analysiert.

**Tabelle 8: Übersicht über die Produktzusammensetzung bei der Umsetzung von Isopulegol (*ee* > 99%) bei Aktivierung des Katalysators mit 1,3-Butandiol.**

| Versuch | Zeit [h] | Umsatz Isopulegol [%]* | Menthon | | Isomenthon | | Selektivität Menthone (gesamt) [%]* | Selektivität Thymol [%]* | Massenbilanz [%] |
|---|---|---|---|---|---|---|---|---|---|
| | | | Selektivität [%]* | *ee* [%] | Selektivität [%]* | *ee* [%] | | | |
| 1 | 5 | 34,3 | 18,2 | > 99 | 11,9 | > 99 | 30,1 | 0 | 93 |
| 2** | 5 | 85,6 | 39,4 | 98,2 | 24,7 | 97,7 | 64,1 | 0,8 | 92 |

*Angaben basierend auf Flächenprozent. **Reaktionstemperatur 190 °C.

Die erzielte Enantioselektivität bei Aktivierung mit 1,3-Butandiol war für die Reaktionsprodukte Menthon und Isomenthon größer als 99%, bei anfänglich geringem Umsatz und geringer Selektivität bei 170 °C. Durch Erhöhung der Temperatur auf 190 °C konnten ab Stunde 6 Umsatz und Selektivität jedoch erhöht werden, bei immer noch hohem ee (> 97 %).

## Patentansprüche

1. Verfahren zur Umsetzung von Isopulegol zu Menthon, wobei Isopulegol in einer Trägergasphase mit einem aktivierten oxidischen Kupferkatalysator, gegebenenfalls enthaltend wenigstens ein weiteres Element ausgewählt unter Aluminium, Mangan, Barium, Chrom, Calcium und Eisen, in Kontakt gebracht wird und gegebenenfalls nach der Umsetzung ein Menthon-haltiges Reaktionsprodukt isoliert wird, wobei die Umsetzung bei einer Temperatur von 150-250°C erfolgt.

2. Verfahren nach Anspruch 1, wobei der Kupferkatalysator mit Wasserstoff oder Wasserstoff und einem Alkohol aktiviert wird.

3. Verfahren nach Anspruch 1 oder 2, wobei im oxidischen Kupferkatalysator Kupfer in der Oxidationsstufe +I oder +II vorliegt.

4. Verfahren nach Anspruch 1 bis 3, wobei das Trägergas Stickstoff, Argon oder ein Gemisch davon umfasst.

5. Verfahren nach einem der vorhergehenden Ansprüche, wobei das eingesetzte Isopulegol ein Enantiomer der Formel I umfasst.

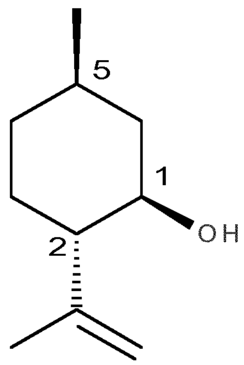

I

6. Verfahren nach einem der vorhergehenden Ansprüche, wobei die Umsetzung bei einer Temperatur von 160-200 °C, insbesondere 160-190 °C, durchgeführt wird.

7. Verfahren nach einem der vorhergehenden Ansprüche, wobei der Kupferkatalysator Kupfer und wenigstens ein weiteres Element ausgewählt unter Aluminium, Mangan, Barium, Chrom, Calcium und Eisen elementar und/oder als Oxide umfasst.

8. Verfahren nach Anspruch 7, wobei die Oxide als Einelementoxide, Doppeloxide und/oder Mehrfachoxide vorliegen.

9. Verfahren nach einem der vorhergehenden Ansprüche, wobei der Kupferkatalysator einen Anteil von mindestens 20% bis zu 100 Gew.-% Kupferverbindung bezogen auf die Trockenmasse des Katalysators aufweist.

10. Verfahren nach einem der vorhergehenden Ansprüche, wobei der Kupferkatalysator ausgewählt ist unter Katalysatoren mit einer der folgenden Zusammensetzungen:

a) 30-40% Kupferoxid, 10-25% Aluminiumoxid, 10-25% Manganoxid und 30-40% Aluminiumkupferoxid
b) 30-45% Kupferoxid, 10-25% Aluminiumoxid, 10-20% Manganoxid und 30-40% Aluminiumkupferoxid
c) 60.0-65.0% Chromkupferoxid ($Cr_2CuO_4$), 20.0-25.0% Kupferoxid, 5.0-10.0% Bariumoxid, 1.0-5.0% Graphit,

1.0% Dichromtrioxid und 1.0% Chromtrioxid,
d) 60.0-70.0% Chromkupferoxid ($Cr_2CuO_4$), 20.0-30.0% Kupferoxid, 1.0-5.0% Mangandioxid, 1.0-5.0% Kieselsäure (Natriumsalz), 1.0-5.0% Graphit und 0.0-0.5 % Kupferchromat,
e) 55.0-65.0% Chromkupferoxid ($Cr_2CuO_4$), 20.0-30.0% Kupferoxid, 5.0-10.0% Siliciumdioxid, 5.0-10.0% Kieselsäure (Natriumsalz) und 1.0-5.0% Graphit,
f) 41.0-46.0% Chromkupferoxid ($Cr_2CuO_4$), 25.0-35.0% Aluminumoxid, 13.0-17.0% Kupferoxid und 10.0-13.0% Bariumchromat
g) 55.0-65.0% Kupferoxid, 25.0-35.0% Calciumsilikat, 5.0-10.0% Palygorskit ($[Mg(Al_{0.5}$-$1Fe_{0-0.5})]$ $Si_4(OH)O_{10.4}H_2O$), 1.0-5.0% Graphit, 1.0-5.0% Siliciumdioxid und 0.5-1.5% Silica (kristallin), und
h) Mischung davon,

jeweils in Gew.-% bezogen auf die Trockenmasse des Katalysators.

**11.** Verfahren nach einem der vorhergehenden Ansprüche, wobei der Katalysator vor der Umsetzung mit Wasserstoff und einem Alkohol gleichzeitig oder zeitlich versetzt in beliebiger Reihenfolge, gegebenenfalls in einem Trägergasstrom, aktiviert wird.

**12.** Verfahren nach Anspruch 11, wobei das Trägergas Stickstoff oder Argon ist und gegebenenfalls bis zu 10 Vol.-% Wasserstoff enthält.

**13.** Verfahren nach einem der vorhergehenden Ansprüche, wobei der für die Aktivierung verwendete Alkohol ausgewählt ist unter gesättigten oder ein- oder mehrfach ungesättigten, geradkettigen oder verzweigten oder cyclischen aliphatischen oder aromatischen Mono- oder Polyolen, insbesondere Mono- oder Diolen, oder Kombinationen davon.

**14.** Verfahren nach einem der vorhergehenden Ansprüche, wobei der für die Aktivierung verwendete Alkohol mindestens eine primäre und/oder sekundäre OH-Gruppe umfasst.

**15.** Verfahren zur Herstellung eines Produktes ausgewählt unter Lebensmitteln, Kosmetika, pharmazeutischen Erzeugnissen, Tabakformulierungen, Haushaltsprodukten und Wäschepflegeprodukten, wobei man einen Menthon-haltigen Zusatz nach einem Verfahren der Ansprüche 1 bis 14 herstellt und diesen Zusatz dem Produkt zugibt.

## Claims

**1.** A process for reacting isopulegol to menthone, in which isopulegol in a carrier gas phase is contacted with an activated oxidic copper catalyst optionally comprising at least one further element selected from aluminum, manganese, barium, chromium, calcium, and or iron, and optionally after the reaction a menthone-containing reaction product is isolated, where the reaction takes place at a temperature of 150-250°C.

**2.** The process according to claim 1, in which the copper catalyst is activated with hydrogen or with hydrogen and an alcohol.

**3.** The process according to claim 1 or 2, in which copper in the oxidic copper catalyst is present in the +I or +II oxidation state.

**4.** The process according to claim 1 to 3, in which the carrier gas comprises nitrogen, argon or a mixture thereof.

**5.** The process according to one of the preceding claims, in which the isopulegol used comprises an enantiomer of the formula I.

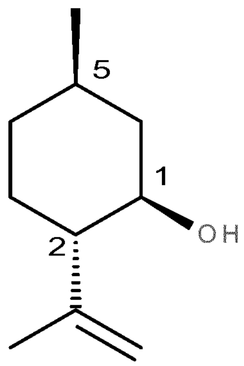

I

**6.** The process according to any of the preceding claims, in which the reaction is carried out at a temperature of 160-200°C, more particularly 160-190°C.

**7.** The process according to any of the preceding claims, in which the copper catalyst comprises copper and at least one further element selected from aluminum, manganese, barium, chromium, calcium, and iron in elemental form and/or as oxides.

**8.** The process according to claim 7, in which the oxides are present as single-element oxides, double oxides and/or multiple oxides.

**9.** The process according to any of the preceding claims, in which the copper catalyst has a fraction of at least 20% up to 100 wt% of copper compound, based on the dry mass of the catalyst.

**10.** The process according to any of the preceding claims, in which the copper catalyst is selected from catalysts having one of the following compositions:

a) 30-40% copper oxide, 10-25% aluminum oxide, 10-25% manganese oxide, and 30-40% aluminum copper oxide,
b) 30-45% copper oxide, 10-25% aluminum oxide, 10-20% manganese oxide, and 30-40% aluminum copper oxide,
c) 60.0-65.0% chromium copper oxide ($Cr_2CuO_4$), 20.0-25.0% copper oxide, 5.0-10.0% barium oxide, 1.0-5.0% graphite, 1.0% dichromium trioxide, and 1.0% chromium trioxide,
d) 60.0-70.0% chromium copper oxide ($Cr_2CuO_4$), 20.0-30.0% copper oxide, 1.0-5.0% manganese dioxide, 1.0-5.0% silicic acid (sodium salt), 1.0-5.0% graphite, and 0.0-0.5% copper chromate,
e) 55.0-65.0% chromium copper oxide ($Cr_2CuO_4$), 20.0-30.0% copper oxide, 5.0-10.0% silicon dioxide, 5.0-10.0% silicic acid (sodium salt), and 1.0-5.0% graphite,
f) 41.0-46.0% chromium copper oxide ($Cr_2CuO_4$), 25.0-35.0% aluminum oxide, 13.0-17.0% copper oxide, and 10.0-13.0% barium chromate,
g) 55.0-65.0% copper oxide, 25.0-35.0% calcium silicate, 5.0-10.0% palygorskite ($[Mg(Al_{0.5}\text{-}1Fe_{0\text{-}0.5})]Si_4(OH)O_{10.4}H_2O$), 1. 0-5. 0% graphite, 1.0-5.0% silicon dioxide, and 0.5-1.5% silica (crystalline), and
h) a mixture thereof,

in each case in wt%, based on the dry mass of the catalyst.

**11.** The process according to any of the preceding claims, in which before the reaction the catalyst is activated with hydrogen and an alcohol, added simultaneously or one before the other, in any order, optionally in a carrier gas stream.

**12.** The process according to claim 11, in which the carrier gas is nitrogen or argon and optionally comprises up to 10 vol% of hydrogen.

**13.** The process according to any of the preceding claims, in which the alcohol used for the activation is selected from saturated or mono- or polyunsaturated, straight-chain or branched or cyclic, aliphatic or aromatic monools or polyols, more particularly monools or diols, or combinations thereof.

14. The process according to any of the preceding claims, in which the alcohol used for the activation comprises at least one primary and/or secondary OH group.

15. A method for producing a product selected from foods, cosmetics, pharmaceutical products, tobacco formulations, household products, and laundry care products, in which a menthone-containing additive is produced by a process of claims 1 to 14 and this additive is added to the product.

**Revendications**

1. Procédé de mise en réaction d'isopulégol pour former de la menthone, selon lequel l'isopulégol est mis en contact dans une phase de gaz vecteur avec un catalyseur à base de cuivre oxydique activé, contenant éventuellement au moins un élément supplémentaire choisi parmi l'aluminium, le manganèse, le baryum, le chrome, le calcium et le fer, et un produit de réaction contenant de la menthone est éventuellement isolé après la réaction, la réaction ayant lieu à une température de 150 à 250 °C.

2. Procédé selon la revendication 1, selon lequel le catalyseur à base de cuivre est activé avec de l'hydrogène ou de l'hydrogène et un alcool.

3. Procédé selon la revendication 1 ou 2, selon lequel le cuivre se présente au niveau d'oxydation +I ou +II dans le catalyseur à base de cuivre oxydique.

4. Procédé selon les revendications 1 à 3, selon lequel le gaz vecteur comprend de l'azote, de l'argon ou un mélange de ceux-ci.

5. Procédé selon l'une quelconque des revendications précédentes, selon lequel l'isopulégol utilisé comprend un énantiomère de formule I

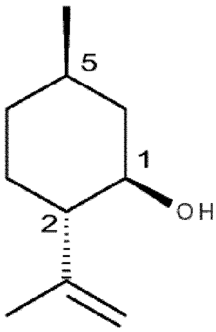

I

6. Procédé selon l'une quelconque des revendications précédentes, selon lequel la réaction est réalisée à une température de 160 à 200 °C, notamment de 160 à 190 °C.

7. Procédé selon l'une quelconque des revendications précédentes, selon lequel le catalyseur à base de cuivre comprend du cuivre et un élément supplémentaire choisi parmi l'aluminium, le manganèse, le baryum, le chrome, le calcium et le fer sous forme élémentaire et/ou sous la forme d'oxydes.

8. Procédé selon la revendication 7, selon lequel les oxydes se présentent sous la forme d'oxydes d'un élément, d'oxydes doubles et/ou d'oxydes multiples.

9. Procédé selon l'une quelconque des revendications précédentes, selon lequel le catalyseur à base de cuivre présente une proportion d'au moins 20 % à 100 % en poids de composé de cuivre par rapport à la masse sèche du catalyseur.

10. Procédé selon l'une quelconque des revendications précédentes, selon lequel le catalyseur à base de cuivre est choisi parmi les catalyseurs ayant les compositions suivantes :

a) 30 à 40 % d'oxyde de cuivre, 10 à 25 % d'oxyde d'aluminium, 10 à 25 % d'oxyde de manganèse et 30 à 40 % d'oxyde d'aluminium et de cuivre,
b) 30 à 45 % d'oxyde de cuivre, 10 à 25 % d'oxyde d'aluminium, 10 à 20 % d'oxyde de manganèse et 30 à 40 % d'oxyde d'aluminium et de cuivre,
c) 60,0 à 65,0 % d'oxyde de chrome et de cuivre ($Cr_2CuO_4$), 20,0 à 25,0 % d'oxyde de cuivre, 5,0 à 10,0 % d'oxyde de baryum, 1,0 à 5,0 % de graphite, 1,0 % de trioxyde de dichrome et 1,0 % de trioxyde de chrome,
d) 60,0 à 70,0 % d'oxyde de chrome et de cuivre ($Cr_2CuO_4$), 20,0 à 30,0 % d'oxyde de cuivre, 1,0 à 5,0 % de dioxyde de manganèse, 1,0 à 5,0 % de silice (sel de sodium), 1,0 à 5,0 % de graphite et 0,0 à 0,5 % de chromate de cuivre,
e) 55,0 à 65,0 % d'oxyde de chrome et de cuivre ($Cr_2CuO_4$), 20,0 à 30,0 % d'oxyde de cuivre, 5,0 à 10,0 % de dioxyde de silicium, 5,0 à 10,0 % de silice (sel de sodium) et 1,0 à 5,0 % de graphite,
f) 41,0 à 46,0 % d'oxyde de chrome et de cuivre ($Cr_2CuO_4$), 25,0 à 35,0 % d'oxyde d'aluminium, 13,0 à 17,0 % d'oxyde de cuivre et 10,0 à 13,0 % de chromate de baryum,
g) 55,0 à 65,0 % d'oxyde de cuivre, 25,0 à 35,0 % de silicate de calcium, 5,0 à 10,0 % de palygorskite ($[Mg(Al_{0,5-1}Fe_{0-0,5})]Si_4(OH)O_{10,4}H_2O$), 1,0 à 5,0 % de graphite, 1,0 à 5,0 % de dioxyde de silicium et 0,5 à 1,5 % de silice (cristalline), et
h) un mélange de celles-ci,

à chaque fois en % en poids par rapport à la masse sèche du catalyseur.

**11.** Procédé selon l'une quelconque des revendications précédentes, selon lequel le catalyseur est activé avant la réaction avec de l'hydrogène et un alcool simultanément ou en décalage dans le temps dans un ordre quelconque, éventuellement dans un courant de gaz vecteur.

**12.** Procédé selon la revendication 11, dans lequel le gaz vecteur est l'azote ou l'argon, et contient éventuellement jusqu'à 10 % en volume d'hydrogène.

**13.** Procédé selon l'une quelconque des revendications précédentes, selon lequel l'alcool utilisé pour l'activation est choisi parmi les mono- ou polyols saturés ou mono- ou polyinsaturés, linéaires ou ramifiés ou cycliques, aliphatiques ou aromatiques, notamment les mono- ou diols, ou les combinaisons de ceux-ci.

**14.** Procédé selon l'une quelconque des revendications précédentes, selon lequel l'alcool utilisé pour l'activation comprend au moins un groupe OH primaire et/ou secondaire.

**15.** Procédé de fabrication d'un produit choisi parmi les produits alimentaires, les produits cosmétiques, les produits pharmaceutiques, les formulations de tabac, les produits ménagers et les produits d'entretien du linge, selon lequel un additif contenant de la menthone est fabriqué par un procédé selon les revendications 1 à 14, et cet additif est ajouté au produit.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 82180463 B
- DE 4236111 A1
- US 4134919 A


### In der Beschreibung aufgeführte Nicht-Patentliteratur


- *Spec. Chem.,* 1987, vol. 7, 193
- *Acta Chem. Scand. B,* 1979, vol. 33, 148
- *J. Org. Chem.,* 1980, vol. 45, 2030
- *Tetrahedron,* 1979, vol. 35, 1789
- **W. TREIBS et al.** *Chem. Ber.,* 1927, vol. 60, 2335
- **FORTI et al.** *Synthesis,* 2001, vol. 1, 52